# EUROPEAN PATENT APPLICATION

(11) **EP 1 757 279 A1**
(43) Date of publication of application: **28.02.2007**
(21) Application number: 06017352.3
(22) Date of filing: 21.08.2006
(51) Int. Cl.: A61K 9/70, A23L 1/00, A23L 1/0524, A61K 31/52

(54) **Dissolvable film**

(30) Priority: 22.08.2005 US 208944
(71) Applicant: National Starch and Chemical Investment Holding Corporation, New Castle, Delaware 19720 (US)
(72) Inventor: Puri, Reema, Bridgewater New Jersey 08807 (US); Zielinski, Richard G., East Brunswick New Jersey 08816 (US)
(74) Representative: Held, Stephan

(57) **Abstract**

Dissolvable films are described for the administration of an active ingredient. The film comprises degraded pectin and at least one other filming forming ingredient. The film can carry actives such as medicaments, flavors, seasonings, scents and the like.

## Description

### FIELD OF THE INVENTION

The invention relates to dissolvable films for the delivery of an active ingredient.

### BACKGROUND OF THE INVENTION

Film compositions that exhibit instant wettability followed by rapid dissolution/disintegration have been used to deliver or administer therapeutic or cosmetic substances, food flavor-imparting agents including food flavorings, or other ingredients contained within the film. Such films generally comprise a water-soluble edible polymer such as, for example, pullulan, starch, pectin and the like. Upon exposure to a moist environment, e.g., the oral cavity, the film dissolves whereby the substance contained therein is released.

Films made of starch are often brittle and/or tacky depending upon the temperature and humidity. Hence, they often have short shelf-life. Although pectin films are not as sensitive to temperature and humidity, its high intrinsic viscosity prevents formation of thin films with high loading levels of actives. Pectin film can also impart uncomfortable or otherwise unsatisfactory mouth-feel during dissolution of the film in the mouth.

There remains a need in the art for rapidly dissolvable films that readily deliver actives with comfortable mouth feel and with low tack or brittleness. The current invention addresses this need.

### SUMMARY OF THE INVENTION

The invention provides a dissolvable film useful in delivering an active ingredient, methods of preparing dissolvable films and methods of using dissolvable films. The dissolvable film comprises an active ingredient present in an amount sufficient to impart a desired action or dose upon administration of a single dosage form of the dissolvable film.

One embodiment of the invention is directed to an active-containing dissolvable film wherein the film comprises a modified pectin, as a film forming ingredient, and at least one other film forming ingredient which is not a modified pectin. In a preferred embodiment, the film forming ingredients comprise, on a dry weight basis, from about 25 to about 75 wt % of a modified pectin and from about 25 to about 75 wt % of the other film forming ingredient, the modified pectin and other film forming ingredient(s) comprising 100% of the film forming ingredients present in the dissolvable film. In a preferred embodiment, the dissolvable films of the invention rapidly dissolves, i.e., completely dissolves in a moist environment within 90 seconds, more preferably within 60 seconds, following exposure to the moist environment, thereby releasing the active into the environment in which it was exposed.

Another embodiment of the invention is directed to a method for making an active-containing dissolvable film comprising forming a mixture of an active ingredient with a modified pectin and at least one other film-forming ingredient, and coating the mixture onto a substrate material to form a film. The film is then dried to a moisture content of less than about 15 weight %, more typically less than about 5 weight % moisture, or less. Alternatively, the actives can be dusted onto the surface of a formed film prior to or during drying. The film is then cut into desired dimensions for the intended end use, packaged, and stored.

Yet another embodiment of the invention is directed to a method of administering an active ingredient to an individual needing or desiring the active. The method comprises applying an active-containing dissolvable film to a moist area of an individual, where after the active is released. The active ingredient is present in the film in an amount sufficient to impart a desired action or to deliver a predetermined dose upon administration of a single dosage form of the active-containing dissolvable film. Ways in which the film may be administered include, but are not limited to, oral administration and topical administration.

### DETAILED DESCRIPTION OF THE INVENTION

Percent by weight means, unless expressly stated otherwise, percent dry weight.

The film composition used in the practice of the invention is not particularly limiting. The composition should be strong, flexible, be blocking and moisture resistant so that it does hot adhere to itself or its packaging, and yet be able to dissolve quickly when placed in a substantially moist, wet or aqueous environment.

The invention provides film compositions which rapidly dissolve in the presence of moisture to release the active ingredient contained therein into the moist environment in which it is placed. The film comprises an active ingredient and at least two different filming forming components, one of which is a modified pectin. The film forming components, if desired contain other optional ingredients.

The film forming components of the rapidly dissolvable film is a mixture of a modified pectin and at least one other film forming ingredient. The modified pectin is typically used in amounts of from about 25 to about 75 dry weight %. The other film forming ingredients, which ingredients are not modified pectin, are also typically used in amounts of from about 25 to about 75 dry weight %, wherein the film forming ingredients (e.g., degraded pectin(s) and other film forming ingredient(s) such as modified starches) equal 100 dry weight % of the film forming ingredients. In one preferred embodiment, the total film forming ingredients comprise a mixture of about 50 dry weight % of a modified pectin and about 50 dry weight% of at least one other film forming ingredient. The mixture of the modified pectin and film forming ingredients allows the initial solution to be prepared at a higher solids loading while keeping the viscosity within manageable limits. It is desirable to have the initial solution at highest solid loading as possible, since less water has to be removed in the drying process and hence, the throughput will be faster. This also leads to a planar film that will curl less. This mixture also forms a film that is more flexible than those made from native pectin alone or native pectin and other film forming ingredient mixtures.

The mixture of modified pectin and other film forming ingredient(s) allows the film to quickly and completely dissolve in a substantially moist or wet environment. The film made from the mixture has instant wettability, which allows the film to soften immediately after the application to, e.g., the tongue, mucosal tissue or the like, thus preventing exposure of the prolonged adverse feeling in the mouth. Due to the quick dissolution from the film, the active agent contained in the film is quickly released.

The term rapidly dissolves means that the film forming component dissolves in less than about 90 seconds, and more preferably dissolves in less than about 60 seconds. It is to be understood that both the film forming ingredients and active may dissolve in the aqueous environment or, alternatively, the film forming ingredients can dissolve and the active released into the aqueous environment, after which it may be swallowed or it may diffuse through the mucosal. The films exhibit moisture and blocking resistance, yet are quickly wetted when exposed to water, such as when placed on the tongue or other substrate surface, followed by rapid dissolution.

The term "substantially aqueous, wet or moist environment" refers to the environment wherein the film forming ingredients dissolve, more preferably rapidly dissolve, releasing the active. Typically, the substantially aqueous environment will be within the oral cavity, e.g., the surface on the tongue, or may be a food product such as a glass of water or juice, soup, bath water or the like. Encompassed are moist environments such as traumatized tissue resulting from a serious burn or the like.

The wettability and dissolution rates of the film may be modified by one skilled in the art to target a specific delivery profile. For example, more rapid dissolution is typically preferred when the film is an oral film while other uses, less rapid dissolution can be tolerated.

An essential component of the film forming composition is modified pectin. It is to be understood that the modified pectin, which comprises from about 25 to about 75 dry weight percent of the film forming ingredient, may comprise of mixture of modified pectins. The modified pectins comprising the mixture may differ in method and/or degree of degradation or other modification.

Pectin that has merely been extracted from its natural source is not considered a modification for the purpose of this invention. Pectin that has been extracted from the cell walls of various fruits, typically by acid treatment, is referred to herein as native pectin.

Modified pectin refers to any change made to native pectin. Included are modifications that would change the viscosity, molecular weight, and/or gelling properties. A degraded pectin, as used herein, is pectin that has been modified to lower its molecular weight, and hence has lower intrinsic viscosity than its original unmodified (native) form. The degraded pectin is formed from the native pectin that has been transformed through physical and/or chemical and/or enzymatic treatments. The degree of degraded pectin is determined by its intrinsic viscosity. Degraded pectin which may be used in the practice of the invention will typically have an intrinsic viscosity of less than about 2.5 dl/g when measured in a 0.05M pH 3.75 citrate buffer system. In contrast, the intrinsic viscosity of pectin prior to modification is about 4.9 dl/g or more when measured under like conditions. While terms, modified pectin and degraded pectin are used interchangeably herein, it is to be understood that modified pectin is not to be limited to degraded pectin.

Some common methods to degrade pectin include mechanical means such as homogenization at high pressures, neutralization of pH with a suitable base or caustic, or with various oxidizing agents. Degraded pectin that can be used in the practice of the invention is commercially available from CP Kelco and is generally sold in 5-30 % soluble solid in water.

Film forming ingredients, as used herein, are water soluble or water dispersible polymers that can form a film upon coating. Water soluble/dispersible solid film forming ingredients conventionally used in the dissolvable film forming art can be used in combination with degraded pectin. Water soluble/dispersible polymers include, but are not limited to starch, modified starch, pullulan, hydroxypropylmethyl cellulose, hydroxyethyl cellulose, hydroxypropyl cellulose, carboxymethyl cellulose, polyvinyl alcohol, polyethylene glycol, tragacanth gum, guar gum, acacia gum, arabic gum, corn fiber gum, polyacrylic acid, methylmethacrylate copolymer, carboxyvinyl polymer, amylose, high amylose starch, hydroxypropylated high amylose starch, dextrin, pectin (i.e., native pectin), chitin, chitosan, levan, elsinan, collagen, gelatin, zein, gluten, soy protein isolate, whey protein isolate, casein, polyvinyl pyrrolidone, sodium alginate, and various mixtures thereof. In one preferred embodiment the film forming ingredients comprise starch and modified pectin, more preferably a modified starch and a degraded pectin.

Starch, as used herein, is intended to include all starches derived from any native source, any of which are suitable for the films of the invention. A native starch, as this term is used herein, is one as it is found in nature. Also suitable are starches derived from a plant obtained by standard breeding techniques including crossbreeding, translocation, inversion, transformation or any other method of gene or chromosome engineering to include variations thereof. In addition, starch derived from a plant grown from artificial mutations and variations of the above generic composition, which may be produced by known standard methods of mutation breeding, are also suitable.

Typical sources for the starches include cereals, tubers, roots, legumes and fruits. The native source can be corn, pea, potato, sweet potato, banana, barley, wheat, rice, sago, amaranth, tapioca, arrowroot, canna, sorghum, and waxy or high amylose varieties thereof. A "waxy" starch is defined as a starch containing at least about 95% by weight amylopectin. A "high amylose" starch is defined as a starch containing at least about 40% by weight amylose. Waxy starch can be waxy maize, waxy rice or waxy barley thereof.

One preferred film forming ingredient is modified starch. The starch may be modified using any modification technique known in the art, including physical and/or chemical and/or enzymatic modifications.

Physically modified starches, such as sheared starches, or thermally-inhibited starches described in the family of patents represented by WO 95/04082, may be used in the practice of the invention.

Chemically modified products are also suitable for use and include, without limitation, those which have been crosslinked, acetylated, organically esterified, cationic, anionic, nonionic, zwitterionic, succinate, and substituted succinate derivatives thereof. Such modifications are known in the art, for example in Modified Starches: Properties and Uses, Ed. Wurzburg, CRC Press, Inc., Florida (1986).

Conversion products derived from any of the starches, including fluidity or thin-boiling starches prepared by oxidation, enzyme conversion, acid hydrolysis, heat and/or acid clextrinization, thermal and/or sheared products may also be useful herein.

Further suitable are pregelatinized starches which are known in the art and disclosed for example in U.S. Patent Nos. 4,465,702, 5,037,929, 5,131,953, and 5,149,799. Conventional procedures for pregelatinizing starch are also known to those skilled in the art and described for example in Chapter XXII- "Production and Use of Pregelatinized Starch", Starch: Chemistry and Technology, Vol. III, Industrial Aspects, R.L. Whistler and E.F. Paschall, Editors, Academic Press, New York 1967.

Starch or starch blends may be purified by any method known in the art to remove off-flavors and colors that are native to the polysaccharide or created during processing. Suitable purification processes for treating starches are disclosed in the family of patents represented by EP 554 818 (Kasica, et al.). Alkali washing techniques, for starches intended for use in either granular or pregelatinized form, are also useful and described in the family of patents represented by U.S. 4,477,480 (Seidel) and 5,187,272 (Bertalan et al.).

Particularly suitable for use are starches capable of emulsifying or encapsulating the active ingredient so that there is no need for additional encapsulating or emulsifying agents, such starches include, without limitation, hydroxyalkylated starches such as hydroxypropylated or hydroxyethylated starches, and succinated starches such as octenylsuccinated or dodecylsuccinated starches. Such starches are commercially available from National Starch and Chemical Company, Bridgewater, NJ, under the tradename VELOX®. The use of emulsifying or encapsulating starches is particularly useful in that a solution or dispersion of the film material (starch component, active agent, and optional additives) may be stored for later processing. The hydroxyalkylated starches have the added advantage of forming a softer film so that there is less or no need for a plasticizer.

The film forming ingredient may be a single modified starch, a blend of modified starches, or a blend of modified and native starches. Blends may be particularly useful to lower the cost of the film or to more easily achieve a variety of desirable properties and functionalities. If native starches are used, they may only be used in minor amounts, particularly less than 15%, more particularly less than about 10% by weight of the starch component.

In addition to the degraded pectin, other film forming ingredients, and the desired active, the films of the invention may also comprise other optional ingredients such as plasticizers, emulsifiers, humectants, surfactants, colorants, proteins, gelling agents, flavors, flavor enhancers sweeteners, and masking agent. It will be appreciated that flavors and/or sweeteners in one embodiment may be an active, while a flavor and/or sweetener in other embodiment may be used, e.g., as a masking agent.

Plasticizer may be added to increase the apparent flexibility of the films. A solid polyol plasticizer will generally provide better resistance to moisture absorption and blocking. One skilled in the art can choose a plasticizer to meet the desired needs of the film, such as choosing an edible plasticizer for an oral film. Plasticizers useful in the instant invention include polyols, polycarboxylic acids, and polyesters. Examples of useful polyols include, but are not limited to ethylene glycol, propylene glycol, sugar alcohols such as sorbitol, manitol, maltitol, lactitol; mono-, di- and oligosaccharides such as fructose, glucose, sucrose, maltose, lactose, and high fructose corn syrup solids and ascorbic acid. Examples of polycarboxylic acids include, but are not limited to, citric acid, maleic acid, succinic acid, polyacrylic acid, and polymaleic acid. Examples of polyesters include but are not limited to glycerin triacetate, acetylated-monoglyceride, diethyl phthalate, triethyl citrate, tributyl citrate, acetyl triethyl citrate, acetyltributyl citrate. More typically, the plasticizers are glycerol, propylene glycol, sorbitol, and/or polyethylene glycol. The plasticizer may be present in any desired amount, particularly from 0 to about 50 percent, more typically from 5 to about 20 by weight of the active containing formulated film.

Other optional components may be added for a variety of reasons including, without limitation, sweeteners, both natural and artificial; emulsifiers such as Polysorbate 80; humectants; surfactants; masking agent such as flavorings; colorants, more particularly food grade colors; proteins such as gelatins; and in addition to flavors, flavor enhancers. Another optional component of the film is a gelling agent. Gelling agents include, but are not limited to carageenan, gellan gums, locust bean, tragacanth gum, guar gum, acacia gum, and arabic gum. These optional components are typically added in minor amounts, particularly less than about 30% total by weight based upon the weight of the final formulated product.

The present invention relates to a film that can be used to administer a desired predetermined substance, referred to herein as an "active", an "active ingredient", an "active agent", and the like, at levels sufficient or effective to impart a desired action or specific intended dose. The term active, also referred to herein as an active ingredient, is used to mean any "drug", "bioactive agent," "preparation," "medicament," "therapeutic agent," "physiological agent," "nutraceutical," "flavor," or "pharmaceutical agent" and include substances for use in the diagnosis, cure, mitigation, arrest, treatment or prevention of a condition or disease state or to affect the structure or function of the body. Dietary supplements, functional foods (e.g., ginger, green tea, lutein, garlic, lycopene, capsaicin, and the like), skin-wellness agents hair fixatives are also included in this term.

Treatment areas where the film of the invention finds use, and examples of pharmaceutical products which can be incorporated in the devices of the invention, include treatment for incontinence (oxybutinin), central nervous system conditions (methylphenidate), hormone therapy and birth control (estradiol, testosterone, progestin, progesterone, levonorgestrel) cardiovascular (nitroglycerin, clonidine) and cardiotonics (e.g., digitalis, digoxin), pain management or anti-inflammatory (fentanyl, lidocaine, diclofenac, flurbiprofen), cosmetic (benzoyl peroxide, salicylic acid, vitamin C, vitamin E, aromatic oils), antinauseants (scopalamine), smoking cessation (nicotine), anti-inflammatory conditions, both steroidal (e.g., hydrocortisone, prednisolone, triamcinolone) and nonsteroidal (e.g., naproxen, piroxicam) treatments, antibacterials (e.g., penicillins such as penicillin V, cephalosporins such as cephalexin, erythromycin, tetracycline, gentamycin, sulfathiazole, nitrofurantoin, and quinolones such as norfloxacin, flumequine, and ibafloxacin), antiprotazoals (e.g., metronidazole), antifungals (e.g. nystatin), calcium channel blockers (e. g. nifedipine, diltiazem), bronchodilators (e.g., theophylline, pirbuterol, salmeterol, isoproterenol), enzyme inhibitors such as collagenase inhibitors, protease inhibitors, elastase inhibitors, lipoxygenase inhibitors, and angiotensin converting enzyme inhibitors (e. g., captopril, lisinopril), other antihypertensives (e.g., propranolol), leukotriene antagonists, anti-ulceratives such as H2 antagonists, antivirals and/or immunomodulators (e.g., 1-isobutyl-1H-imidazo[4,5-c] quinolin-4-amine, 1-(2-hydroxy-2-methyl-propyl)-1 H-imidazo[4,5-c] quinoline-4-amine, and acyclovir), local anesthetics (e.g., benzocaine, propofol), antitussives (e.g., codeine, dextromethorphan), antihistamines (e.g., diphenhydramine, chlorpheniramine, terfenadine), narcotic analgesics (e.g., morphine, fentanyl), cardioactive products such as atriopeptides, anticonvulsants (e.g., carbamazine), immunosuppressives (e. g., cyclosporine), psychotherapeutics (e.g., diazepam), sedatives (e.g., phenobarbital), anticoagulants (e.g., heparin), analgesics (e.g., acetaminophen), antimigrane agents (e.g., ergotamine, melatonin, sumatriptan), antiarrhythmic agents (e.g., flecainide), antiemetics (e.g., metaclopromide, ondansetron), anticancer agents (e.g., methotrexate), neurologic agents such as anxiolytic drugs, hemostatics, anti-obesity agents, and the like, as well as pharmaceutically acceptable salts, esters, solvates and clathrates thereof. Additional examples of active include, but are not limited to guaifenesin, loratidine, L-theanine, ompremazole, pseudoephedrine hydrochloride, and vitamins like niacin or retinol.

Veterinary actives may also be administered using the films of the invention. The films may be administered to domestic and non-domestic animals. Films for veterinary administration may administer, for example, a breath freshening agent, flavor, dietary supplement, functional food, pharmaceutical, and/or nutraceutical active. In addition to many of the above mentioned actives, which can also be used in veterinary applications, additional examples include e.g., diclazuril and lufenuron.

Nutraceutical actives may also be administered using the film of the invention. Common nutraceuticals include, but are not limited to functional foods, dietary supplements, herbal products, including anti-oxidants, immune enhancers, cardiovascular health enhancers, healthy joints and cartilage enhancers, memory and mental enhancers, women's health enhancers, mood and emotional enhancers, and weight loss enhancers. These include, but not are not limited to caffeine, folic acid, beta-carotene, lycopene, valerian, ginseng, vitamin E, herbal teas (e.g., green tea), and natural biological flora.

Flavorings can also be administered using the film of the invention. These flavorings may be chosen from synthetic flavor oils and flavoring aromatics, and/or oils, oleo resins and extracts derived from plants, leaves, flowers, fruits and so forth, and combinations thereof. Representative flavor oils include: spearmint oil, cinnamon oil, peppermint oil, clove oil, bay oil, thyme oil, cedar leaf oil, oil of nutmeg, oil of sage, and oil of bitter almonds. Also useful are artificial, natural or synthetic fruit flavors such as vanilla, chocolate, coffee, cocoa and citrus oil, including lemon, orange, grape, lime and grapefruit and fruit essences including apple, pear, peach, strawberry, raspberry, cherry, plum, pineapple, apricot and so forth. These flavorings can be used individually or in admixture. Commonly used flavors include mints such as peppermint, artificial vanilla, cinnamon derivatives, and various fruit flavors, whether employed individually or in admixture. Flavorings such as aldehydes and esters including cinnamyl acetate, cinnamaldehyde, citral, diethylacetal, dihydrocarvyl acetate, eugenyl formate, p-methylanisole, and so forth may also be used. Generally, any flavoring or food additive, such as those described in Chemicals Used in Food Processing, publication 1274 by the National Academy of Sciences, pages 63-258, may be used. Further examples of aldehyde flavorings include, but are not limited to acetaldehyde (apple); benzaldehyde (cherry, almond); cinnamic aldehyde (cinnamon); citral, i.e., alpha citral (lemon, lime); neral, i.e. beta citral (lemon, lime); decanal (orange, lernon); ethyl vanillin (vanilla, cream); heliotropine, i.e., piperonal (vanilla, cream); vanillin (vanilla, cream); alpha-amyl cinnarnaldehyde (spicy fruity flavors); butyraldehyde (butter, cheese); valeraldehyde (butter, cheese); citronellal (modifies, many types); decanal (citrus fruits); aldehyde C-8 (citrus fruits); aldehyde C-9 (citrus fruits); aldehyde C-12 (citrus fruits); 2-ethyl butyraldehyde (berry fruits); hexenal, i.e. trans-2 (berry fruits); tolyl aldehyde (cherry, almond); veratraldehyde (vanilla); 2,6-dimethyl-5-heptenal, i.e. melonal (melon); 2-6-dimethyloctanal (green fruit); and 2-dodecenal (citrus, mandarin); cherry, grape; mixtures thereof, and the like.

The amount of flavoring employed is normally a matter of preference subject to such factors as flavor type, individual flavor, strength desired and taste masking required. Thus, the amount may be varied in order to obtain the result desired in the final product. Such variations are within the capabilities of those skilled in the art without the need for undue experimentation.

Preferred for delivery in accordance with the invention are actives that are water soluble at some level, and includes actives that can only be solubilized within an aqueous environment upon agitation, upon the application of heat, upon a change in pH, or upon application of heat and/or agitation and/or pH change. In one particularly preferred embodiment the active for incorporation into and delivery by means of the dissolvable film of the invention is caffeine.

The active is added in such amounts such that the final active-containing single dosage dissolvable film comprises a predetermined effective amount. By sufficient level or amount is meant that the active agent is present in amounts required to impart a desired action or therapeutic dose, such as a desired organoleptic, physiological, therapeutic, nutraceutical, or flavor effect. The active is present in an amount sufficient, also referred to herein as an effective amount, to bring about a desired result, e.g., a desired therapeutic result in the treatment of a condition. An effective amount of a drug, for example, means a nontoxic but sufficient amount of a drug to provide the selected effect over a specific period of time or number of doses.

It will be appreciated that an amount that constitutes a effective amount will vary according to the particular active incorporated in the film, the condition and/or severity of the condition being treated, any other actives being co-administered with the selected active, other components of the film, desired duration of treatment and preferred number of dosage units, age of the individual to which the film is being administered, the size of the film, and the like. Generally the active can comprise up to 33 %, or even more, by weight of the finally formulated film. Pharmaceutical actives typically range from about 1 to 15 weight %; and functional food and nutraceutical actives typically range from about 10-33 weight % of the finally formulated film. Such an amount is readily determinable by the skilled practitioner.

The action exerted by the active may be perceived and measured in two ways. The level of active that is sufficient to impart a desired action can be measured by its perceived intensity and its perceived character. Intensity is defined as the overall strength of e.g., the taste, the smell or physiological reaction (e.g., strong, moderate, weak or slight, etc.). Character is defined as the perceived description of the of e.g., the taste, smell, physiological or pharmaceutical reaction (chocolate, peppermint, citrus, heightened alertness, amelioration of symptoms of disease or injury, and the like)

The dissolvable film of the invention may be made by a variety of processes known in the art. In the practice of the invention, the active is solubilized or dispersed in an aqueous environment at or above room temperature. The active may be first solubilized or dispersed in water, and then the active-containing solution or suspension is mixed with the degraded pectin and film forming ingredients to form a mixture. Alternatively, and more preferably, the active may be solubilized or dispersed in a solution of the film forming ingredients to form a mixture.

Films may be formed from such dispersions or solutions by shaping it into a solidified form of a suitable thickness by any technique known in the art including, but not limited to, wet casting, freeze-drying, and extrusion molding. The dispersion or solution may also be directly coated or sprayed onto another edible product, such as a tablet or foodstuff, and dried to form an edible film.

The prepared active and film forming containing mixture is applied to a substrate, using knife, bar or extrusion die coating methods, drying the coated substrate to remove the majority of the water, and removing the film from the substrate. Suitable substrates include, but are not limited to, silicone elastomers, metal foils and metalized polyfoils, composite foils or films containing polyethylene terephthalate (PET), polytetrafluoroethylene materials or equivalents thereof, polyether block amide copolymers, polyurethanes, polyvinylidene chloride, nylon, polyethylene, polyester, and other such materials useful in the art as releasable substrates.

The film is not completely dried in that some degree of water remains. The amount of water may be controlled to obtain desired functionality. For example, more water typically results in a more flexible film, while too much water results in a film that will block (i.e., stacked films will adhere to one another and be difficult to separate) and be tacky. Typically, the films of the invention will have a moisture content of less than about 15 weight % moisture, preferably from about 5 weight % to about 15 weight % moisture, even more preferably from about 6 weight % to about 10 weight % moisture. The film may then be cut to the desired dimension, packaged and stored.

The film thickness will depend, in part, on the desired end use. Typically, the film thickness will be in the range of about 10 to 500 microns, particularly 25 to 200 microns. When prepared as an oral film for quick dissolution in the oral cavity, the film thickness is more preferably from about 50 to 150 microns.

The films of the invention can be made in the form of an article such as a strip, tape, a patch, a sheet, a dressing or any other form known to those skilled in the art. The dosage system may be produced in any desirable unit form. In addition to having various shapes, the dosage units produced may come in various sizes depending on the end use application (e.g., whether designed for oral or topical administration).

Generally the device will be in the form of a strip of a size suitable to deliver a pre-selected amount of drug into the oral cavity without bending or folding the film. The thickness may vary over a wide range, typically from about 1 to about 5 mils, preferably from about 3 to about 5 mil thick, more typically from about 4 to about 5 mil thick. Generally a strip of about 1 inch in width, about 1¼ inch in length and about 4 mil in thickness (about 105 mg by weight) will be used for oral administration.

In one embodiment, the invention provides a strip of rapidly dissolvable film containing at least about 10 % by dry weight of caffeine as the active substance. In a preferred embodiment, the prepared film comprises at least about 15 % by dry weight of caffeine, based on the weight of the finally formulated film. Caffeine can comprise up to about 33 % by weight or more of the finally formulated film.

A further embodiment of the invention is directed to administering an active ingredient to an individual needing or desiring said active ingredient. The method comprises applying an active-containing dissolvable film to a moist area of an individual, such as the tongue or skin, upon which application the active is released. It will be appreciated that, in terms of compliance, the films of the invention are particularly useful in treating young children.

The films may be used for delivering any active agent for a variety of applications including personal care, skin care, wound care, pharmaceutical, veterinarian, nutraceutical, and breath freshener.

The films of the invention may be stacked for multi-dose packaging or, if desired, be packaged in single dose form. A single dosage form will typically be a single film, e.g., a strip of film formulated for oral delivery, but may refer to multiple films administered at substantially the same time. In this regard it is common in the medicinal art that one or two tablets are recommended, e.g., depending on body weight, age, or the like, for administration as a single dose.

One skilled in the art can also modify the film formulation to provide clarity and other desired characteristics by manipulation of the actives, degraded pectin and film forming ingredient, and control of other components.

The following examples are presented for purpose of illustration only.

### EXAMPLES

### EXAMPLE 1

### Composition and Method of Forming the Film

Table I and II list the components and their respective dry weight percent to form the rapidly dissolvable films. Table I lists the ingredients to form a caffeine film with 50% degraded pectin, and Table II is directed to a caffeine film with 25% degraded pectin in the film forming component. Both formed non-brittle, non-tacky film with good mouth feel that completely dissolved in the mouth under 60 seconds.

All ingredients were weighed out and blended. The ingredients and their respective amounts (in weight and dry weight percents) are listed in following Tables. Gellan gum and plasticizers (propylene glycol, glycerol, sorbitol) were combined in a beaker for approximately 15 minutes to obtain a smooth solution. Flavors (peppermint, vanilla mint) and Polysorbate 80 were then added to the gellan gum solution. In Waring Commercial Laboratory Blender, model 34BL97, the rest of the ingredients (Velox® starches, sucralose, Ace K, caffeine, Blue 1, Genu pectin) along with the gellan gum solution and water were added. This was blended at speed setting three for 3-5 minutes until the solution becomes homogenous.

**Table I**

| Ingredient | Manufacturer | Weight (g) | % dry weight |
|---|---|---|---|
| Genu Pectin - Type D slowest (11.5 weight % solids) | CP Kelco | 67.00 | 19.26 |
| Velox® starches | National Starch & Chemical | 7.70 | 19.25 |
| Caffeine | Spectrum | 8.00 | 20.00 |
| Gellan gum | CP Kelco | 0.12 | 0.30 |
| Propylene Glycol | Spectrum | 1.00 | 2.50 |
| Glycerol | Spectrum | 3.70 | 9.25 |
| Sorbitol | Spectrum | 1.10 | 2.75 |
| Peppermint | Quest | 1.71 | 4.27 |
| Vanilla mint | Quest | 3.05 | 7.62 |
| Blue 1 | Quest | 0.02 | 0.05 |
| Polysorbate 80 | Spectrum | 1.30 | 3.25 |
| Sucralose | McNeil-PPC | 2.30 | 5.75 |
| Ace K | Spectrum | 2.30 | 5.75 |
| Water | | 20.70 | |

**Table II**

| Ingredient | Manufacturer | Weight (g) | % dry weight |
|---|---|---|---|
| Genu Pectin Type D slowest (11.5 weight % solids) | CP Kelco | 33.48 | 9.64 |
| Velox® starches | National Starch & Chemical | 11.50 | 28.79 |
| Caffeine | Spectrum | 8.00 | 20.03 |
| Gellan gum | CP Kelco | 0.12 | 0.30 |
| Propylene Glycol | Spectrum | 1.00 | 2.50 |
| Glycerol | Spectrum | 3.70 | 9.26 |
| Sorbitol | Spectrum | 1.10 | 2.75 |
| Peppermint | Quest | 1.71 | 4.28 |
| Vanilla mint | Quest | 3.05 | 7.63 |
| Blue 1 | Quest | 0.02 | 0.05 |
| Polysorbate 80 | Spectrum | 1.30 | 3.25 |
| Sucralose | McNeil-PPC | 2.30 | 5.76 |
| Ace K | Spectrum | 2.30 | 5.76 |
| Water | | 30.42 | |

### EXAMPLE 2

### Method of Forming the Caffeine Film

To cast a film, 10 cc of the blended caffeine film solution was pulled with a syringe. The solution was then ejected onto the coater to cast a film on a PET substrate. The wet film was initially casted at 3.5 mils (89 microns). This film on the substrate was then dried in a 150°F convection oven for 5-8 minutes, or until not tacky to touch. The final film dried to 2.5 - 3.0 mil (64-76 micron) thickness. This was cut into appropriate dimensions, packed, and stored.

Many modifications and variations of this invention can be made without departing from its spirit and scope, as will be apparent to those skilled in the art. The specific embodiments described herein are offered by way of examples only, and the invention is to be limited only by the terms of the appended claims, along with the full scope of equivalents to which such claims are entitled.

## Claims

1. A dissolvable film comprising a mixture of film forming ingredients, said mixture comprising from about 25 to about 75 dry weight % of a modified pectin.

2. The film of claim 1 further comprising an active ingredient.

3. The dissolvable film of claim 2 wherein said film completely dissolves within 60 seconds after exposure to moisture.

4. The film of claim 2 wherein said active ingredient is a pharmacological, nutraceutical, dietary supplement, functional food, cosmetic, flavor or mixture thereof.

5. The film of claim 2 which comprises from about 10 to about 33 %, based on the final formulated weight of the film, of caffeine.

6. The film of claim 1 wherein said modified pectin is derived from pectin by means of physical, chemical or enzymatic treatment.

7. The film of claim 1 wherein said mixture comprises from about 25 to about 75 dry weight % of a film forming ingredient other than a modified pectin.

8. The film of claim 7 wherein the other filming forming ingredient is selected from the group consisting of starch, modified starch, pullulan, hydroxypropylmethyl cellulose, hydroxyethyl cellulose, hydroxypropyl cellulose, carboxymethyl cellulose, polyvinyl alcohol, polyethylene glycol, tragacanth gum, guar gum, acacia gum, arabic gum, corn fiber gum, polyacrylic acid, methylmethacrylate copolymer, carboxyvinyl polymer, amylose, high amylose starch, hydroxypropylated high amylose starch, dextrin, pectin, chitin, chitosan, levan, elsinan, collagen, gelatin, zein, gluten, soy protein isolate, whey protein isolate, casein, polyvinyl pyrrolidone, sodium alginate, and combinations thereof.

9. The film of claim 8 wherein at least one of the other film forming ingredient is a modified starch.

10. The film of claim 9 wherein the modified starch is selected from the group consisting of hydroxymethylated starch, hydroxyethylated starch, hydroxypropylated starch, octenylsuccinated starch, and dodecylsuccinated starch, and combinations thereof.

11. The film of claim 1 wherein the film further comprises one or more plasticizers.

12. The film of claim 1 wherein the film further comprises a component selected from the group consisting of sweeteners, emulsifiers, humectants, surfactants, colorants, flavor enhancers, gelling agents, and mixtures thereof.

13. The film of claim 1 wherein said mixture comprises about 50 dry wt % of a modified pectin and about 50 dry wt % of at least one film forming ingredient other than modified pectin.

14. The film of claim 13 further comprising an active ingredient.

15. The film of claim 14 wherein the active ingredient is caffeine.

16. The film of claim 14 wherein said other film forming ingredient is a combination of modified starches.

17. A method of administering an active ingredient to an individual needing or desiring said active ingredient comprising applying the active-containing dissolvable film of claim 2 to a moist area of an individual, where the active is released.

18. The method of claim 17, wherein the film is applied to the tongue.

19. The method of claim 17, wherein the film is applied to traumatized tissue.

20. The method of claim 18, wherein the active is caffeine.
